Europäisches Patentamt

European Patent Office

Office européen des brevets

(11) Numéro de publication: **0 296 050**
**A1**

## (12) DEMANDE DE BREVET EUROPEEN

(21) Numéro de dépôt: 88401463.0

(22) Date de dépôt: 14.06.88

(51) Int. Cl.⁴: **G 01 N 33/53**
G 01 N 33/543, G 01 N 33/68

(30) Priorité: 16.06.87 FR 8708383

(43) Date de publication de la demande:
21.12.88 Bulletin 88/51

(84) Etats contractants désignés:
AT BE CH DE ES FR GB GR IT LI LU NL SE

(71) Demandeur: IRE MEDGENIX S.A.
B-6220 Fleurus (BE)

(72) Inventeur: **Delacroix, Dominique**
**Avenue des Ramiers, 11**
**B-1950 Kraainem (BE)**

**Gevaert, Yvonne**
**Rue Lagrange, 27**
**B-4050 Anthisnes (BE)**

**Reuter, Aimée**
**Boulevard Frère Orban, 15 Bte 022**
**B-4000 Liège (BE)**

**Wantier, Henri**
**Rue d'Italie, 31**
**B-7370 Elouges (BE)**

(74) Mandataire: **Warcoin, Jacques et al**
**Cabinet Régimbeau 26, avenue Kléber**
**F-75116 Paris (FR)**

(54) Solution d'une substance de nature polypeptidique, et utilisation pour des dosages immunologiques.

(57) La présente invention concerne une solution d'une première substance de nature polypeptidique, utile notamment pour réduire l'adsorption sur phase solide et la fixation non spécifique de ladite première substance.

Selon la présente invention, on ajoute à la solution une seconde substance choisie parmi les amino-acides basiques monomères ou polymérisés.

L'invention concerne également une trousse de dosages immunologiques qui comporte des flacons contenant ladite première substance antigène et/ou anticorps marquée ou non dans des solutions avec ladite seconde substance ou des lyophilisats de ces solutions.

EP 0 296 050 A1

**Description**

## SOLUTION D'UNE SUBSTANCE DE NATURE POLYPEPTIDIQUE ET UTILISATION POUR DES DOSAGES IMMUNOLOGIQUES

La présente invention concerne une solution d'une substance de nature polypeptidique, utile notamment pour réduire les phénomènes d'adsorption de ladite substance.

Dans la présente demande, on entend par phénomènes d'adsorption, aussi bien

1) l'adsorption sur phase solide que

2) des phénomènes de fixation non spécifique à d'autres molécules.

On entend par fixation non spécifique une fixation autre qu'avec l'anticorps ou l'antigène spécifiques lorsque la substance est un antigène ou un anticorps.

Les substances polypeptidiques sont en généraldans des solutions tampons comprenant entre autres des agents stabilisateurs du type protéique, principalement de l'albumine de sérum de bovin (ou BSA).

Cependant, on a découvert, selon la présente invention, que de nombreuses substances de nature polypeptidique n'étaient pas stabilisées de façon suffisante par ces agents stabilisateurs tels que la BSA et qu'il apparaissait des phénomènes d'adsorption du type de ceux précités, phénomènes très dommageables en cas notamment de dosages immunologiques de la substance.

Ainsi, vraisemblablement, en vertu de leur structure non glycosylée, de nombreuses protéines de recombinants génétiques présentent des phénomènes d'adsorption sur phase solide et de fixation non spécifique très importants, même conservés dans des solutions stabilisées à la BSA. Cet état de fait est particulièrement sensible dans le cas de l'interféron $\gamma$ (IFNY$\gamma$) préparé par génie génétique comme nous le verrons par la suite (exemple 1). Cette adsorption sur phase solide se produit également pour des protéines naturelles purifiées, comme pour la myéloperoxydase.

Cette adsorption sur phase solide a lieu sur les parois internes du flacon de conservation ou des tubes de dosages des substances. Elle se traduit alors par une perte d'immunoréactivité importante de la substance qui affecte considérablement la qualité de son dosage immunologique. Cette perte d'immunoréactivité est observée pour des traceurs et des standards conservés dans des flacons à 4°C, surtout après avoir été congelés et lyophilisés.

Cette adsorption sur phase solide a également des conséquences fâcheuses lorsqu'elle apparaît dans les colonnes de purification. Ceci est le cas, après le marquage radioactif de l'antigène ou de l'anticorps, pour la préparation du traceur en vue de dosages immunologiques, et peut conduire à des rendements très mauvais de purification dudit traceur.

Outre ce phénomène d'adsorption sur phase solide, on a donc également constaté des phénomènes relevant plutôt de la fixation à d'autres molécules. En particulier, on a constaté lors de dosages immunologiques la fixation de certains antigènes sur les anticorps non spécifiques utilisés pour précipiter le complexe(antigène marqué - anticorps spécifique),fixation non spécifique qui s'accompagne aussi d'une perte d'immunoréactivité de la substance et en affecte le dosage immunologique.

Le but de la présente invention était donc de remédier à ces inconvénients en proposant une solution à ces problèmes d'adsorption de certaines substances de na ture polypeptidique, solution qui préserve néanmoins la sensibilité et la spécificité de la réaction (antigène-anticorps spécifique)en vue de dosages immunologiques.

Pour ce faire, la présente invention propose d'ajouter dans les solutions classiques de dilution de ces substances, telles que les solutions tampons, un amino-acide basique monomère ou polymérisé.

On a découvert en effet qu'en ajoutant dans les tampons de dilution d'une substance de nature polypeptidique un amino-acide basique monomère ou polymérisé, à savoir de la lysine ou polylysine, de l'arginine ou polyarginine et de l'histidine ou polyhistidine, on réduisait de façon remarquable les phénomènes d'adsorption - pouvant intervenir sur phase solide, comme le verre ou divers polymères (polypropylène, polystyrène).

Cette spécificité des amino-acides basiques est vraisemblablement liée à la présence dans leur structure moléculaire d'un groupe amine supplémentaire par rapport aux autres amino-acides, qui permet peut-être à ces substances amino-acides basiques de s'adsorber préférentiellement à la substance à protéger, sur phase solide.

Ainsi, on a remarqué qu'en ajoutant en particulier de la polylysine par exemple, dans les tampons d'élution classiques des antigènes marqués (traceurs) pour leur purification à travers une colonne du type Séphadex, on obtenait des rendements de purification nettement meilleurs.

On a aussi constaté qu'en ajoutant de la polylysine dans les tampons classiques de conservation du traceur avant dosage et les tampons d'incubations classiques du traceur, de l'antigène à doser ou de l'antisérum lors du dosage proprement dit, on obtenait une meilleure immunoréactivité due à une réduction de l'adsorption sur les parois de flacons et tubes de dosage et une fixation très faible avec les anticorps non spécifiques de précipitation. Ceci offre de surcroît la possibilité d'utiliser le traceur pour un dosage immunologique sans le purifier au préalable sur une colonne Séphadex.

Cette diminution de l'adsorption sur phase solide et de la fixation non spécifique est particulièrement notable dans le cas de polypeptides préparés par génie génétique, notamment pour l'IFNY$\gamma$ comme évoqué ci-dessus, mais aussi pour d'autres cytokines, comme les interleukines 1 et 2 ou le tumor nécrosing factor

(TNFα) et encore pour d'autres substances polypeptidiques telles que le glucagon, l'insuline ou la myéloperoxydase.

La présente invention a donc pour objet des solutions d'une première substance de nature polypeptidique, visant notamment à réduire les phénomènes d'adsorption de ladite première substance, caractérisées en ce que la solution comporte outre ladite première substance,notamment une seconde substance choisie parmi les amino-acides basiques monomères ou polymérisés.

Parmi ces amino-acides basiques monomères ou polymérisés, on peut citer particulièrement la polylysine.

Les résultats les plus satisfaisants ont été obtenus avec de la polylysine de poids moléculaire 14 000 et de degré de polymérisation 70.

On peut ajouter ces secondes substances dans des faibles dilutions pouvant descendre jusqu'à 0,05 %o et même jusqu'à 0,01 %o. Cette caractéristique est particulièrement intéressante,compte tenu du prix très élevé de ces produits.

La solution peut être un tampon d'élution classique pour la purification de la substance de nature polypeptidique marquée ou non à travers une colonne de purification, tampon auquel on a rajouté un amino-acide basique monomère ou polymérisé.

La solution peut également être un tampon de conservation ou d'incubation de la substance de nature polypeptidique marquée ou non, antigène ou anticorps, destiné à des dosages immunologiques, la phase solide étant la paroi interne du flacon ou des tubes de dosage.

Ces tampons peuvent être des tampons phosphate ou des tampons Tris, par exemple.

On peut citer entre autres un tampon phosphate 0,05 M pH 7,5 avec 5 %o de BSA et 0,5 %o de NaN$_3$ et éventuellement 5 à 20 % de polyéthylène glycol.

On peut citer également, comme tampon Tris, un tampon Tris 40 mM pH 8 avec 30 mM de NaCl, 20 % de glycérol, 10 mM de dithiothréitol avec éventuellement 5 à 20 % de polyéthylène glycol.

Enfin, un avantage supplémentaire des solutions conformes à l'invention est qu'elles permettent éventuellement de conserver lesdites premières substances sans agent stabilisateur du type protéique tel que la BSA. Cet avantage est très intéressant lorsqu'on envisage le marquage de ladite première substance, car l'agent stabilisateur, généralement présent dans la solution, se marque également, ce qui n'est pas le cas de la deuxième substance amino-acide basique.

La présente invention a également pour objet une trousse de réactifs en vue de dosages immunologiques comportant des flacons contenant ladite première substance antigène et/ou antisérum,marquée ou non,dans des solutions conformes à la présente invention ou sous forme de lyophilisats desdites solutions.

Notamment, la présente invention a pour objet une trousse de dosages immunologiques d'une substance choisi parmi l'interféron γ, l'interleukine 1 et la mycloperoxydase comportant des flacons contenant ladite substance ou un antisérum dirigé contre ladite substance,marqués ou non, en solution dans un tampon comportant un amino-acide basique monomère ou polymérisé, notamment de la polylysine, et lyophilisés à partir de telles solutions.

Comme il a déjà été évoqué précédémment et comme il sera explicité dans la suite de la description, l'apport de ladite seconde substance est particulièrement avantageux lorsqu'il y a conservation de ladite première substance sous forme lyophilisée.

Il s'agit ici de dosage d'antigènes ou d'anticorps en solution tels qu'ils existent dans les liquides biologiques. Il existe une très grande variété de principes qui ont donné naissance à des trousses de réactifs commercialisés en vue de ces dosages.

Les schémas réactionnels sont nombreux et peuvent faire l'objet de multiples classements particu liers en fonction de :

- a) la nature de la substance à doser (antigène ou anticorps),

- b) le mode réactionnel, compétitif ou non compétitif. Par exemple, si l'on met en présence d'une quantité définie d'anticorps spécifique une quantité également définie d'antigène marqué (traceur) et une quantité inconnue d'antigène normal non marqué et à doser, les molécules d'antigène marquées et non marquées vont se trouver en compétition à l'égard de l'anticorps. Au contraire, d'autres modes réactionnels se font à partir de réactions successives de sorte qu'il n'y a pas de compétition. On peut aussi fixer des anticorps spécifiques sur un support solide, faire réagir ensuite l'antigène à doser sur ces anticorps insolubilisés. Si cet antigène a au moins deux sites, il peut encore réagir avec une seconde molécule d'anticorps qui sera marquée. Dans un tel cas, il n'y a pas compétition. On peut aussi faire réagir simultanément l'antigène à doser et l'antigène froid (standard) ainsi que la seconde molécule d'anticorps marquée, de cette façon il y aura compétition.

- c) la méthodologie utilisée pour séparer l'antigène (à doser) se trouvant sous forme de complexe avec l'anticorps (dit "lié") de l'antigène n'ayant pas réagi (dit "libre"). On peut, bien entendu, retourner la proposition et parler d'anticorps au lieu d'antigène.

Les avantages et caractéristiques de la présente invention seront maintenant plus explicités à l'aide des exemples suivants.

Ces exemples font référence à des dessins sur lesquels :

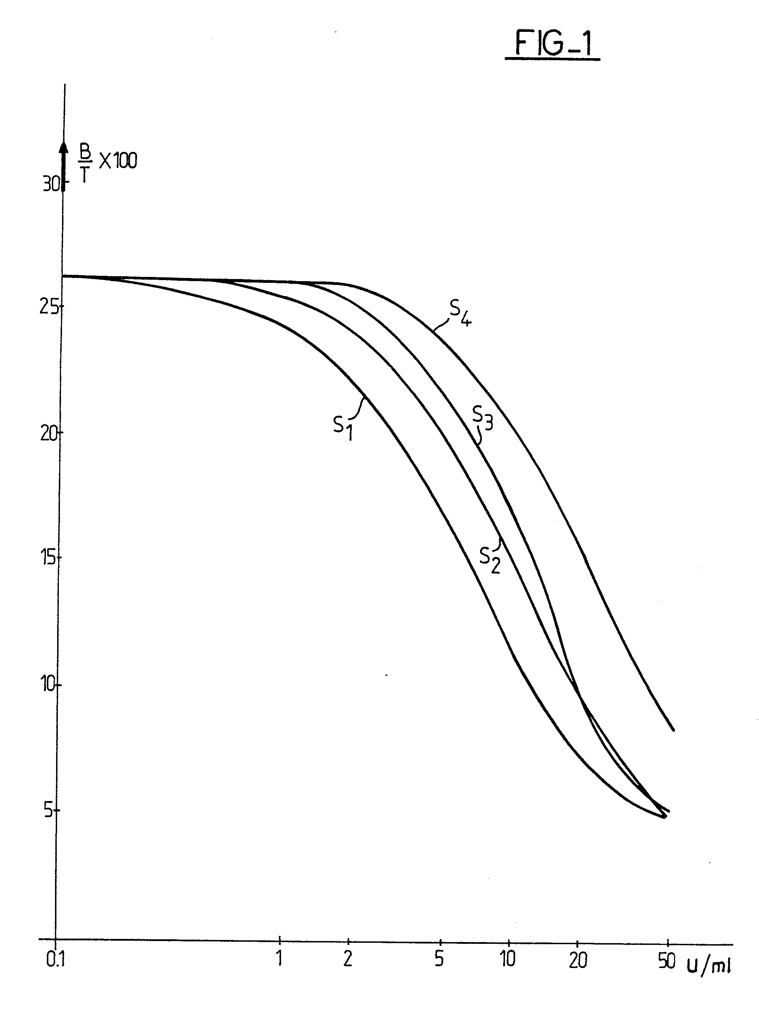

. la figure 1 représente des courbes standard de dosages pour un même interféron γ conservé dans différentes conditions sans polylysine,

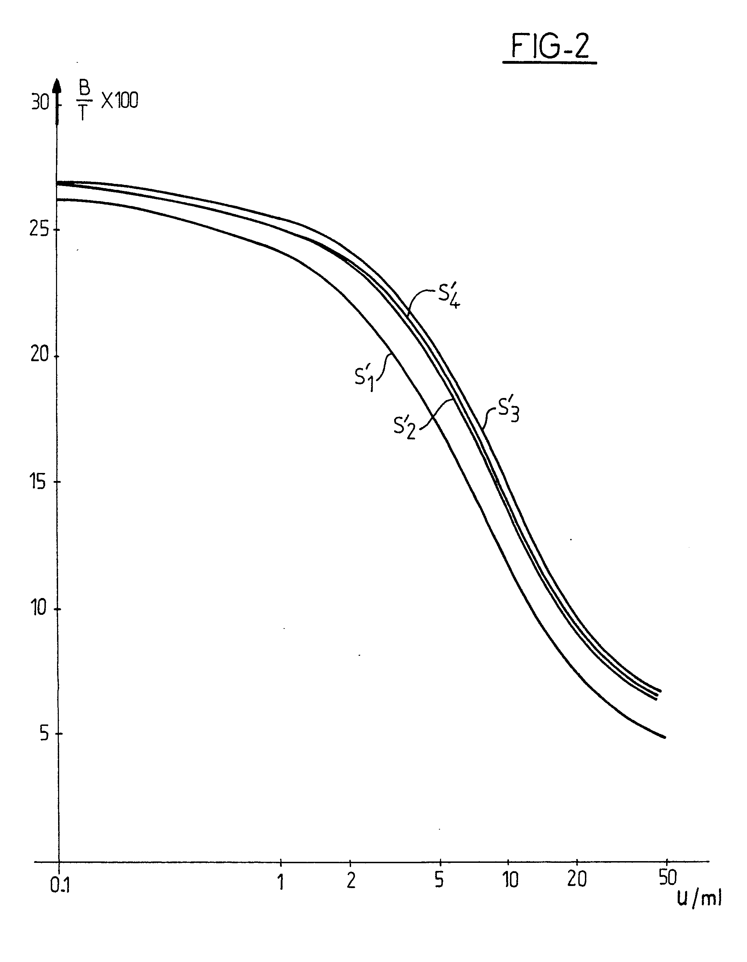

. la figure 2 représente des courbes standard de dosages RIA pour l'IFNγ conservé de différentes façons en "tampon polylysine" avant l'incubation du dosage,

3

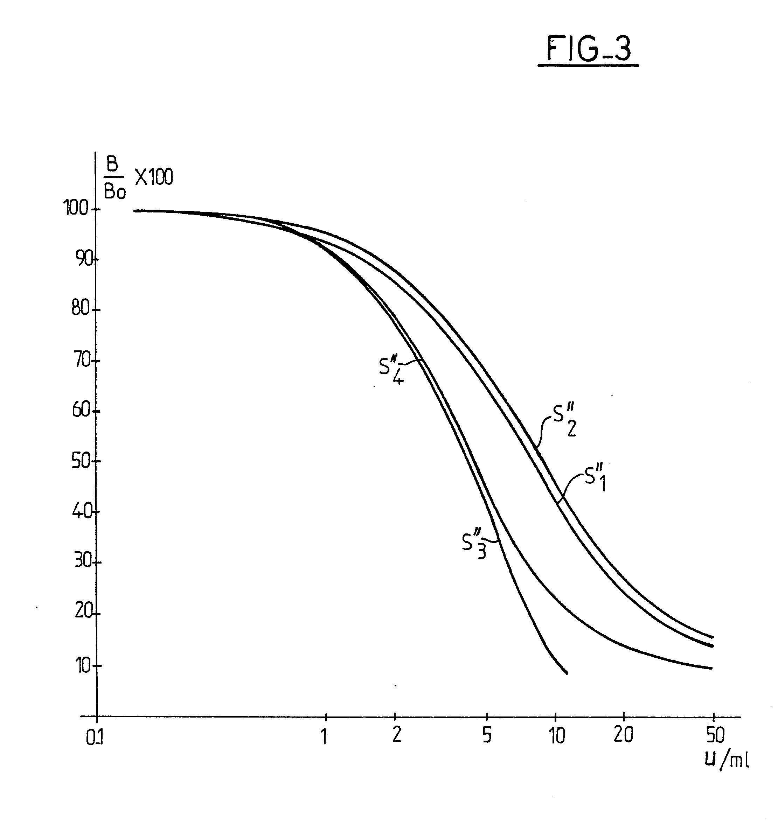

. la figure 3 représente des courbes standard RIA pour différents IFNγ avec et sans "tampon polylysine" à l'incubation lors du dosage seulement,

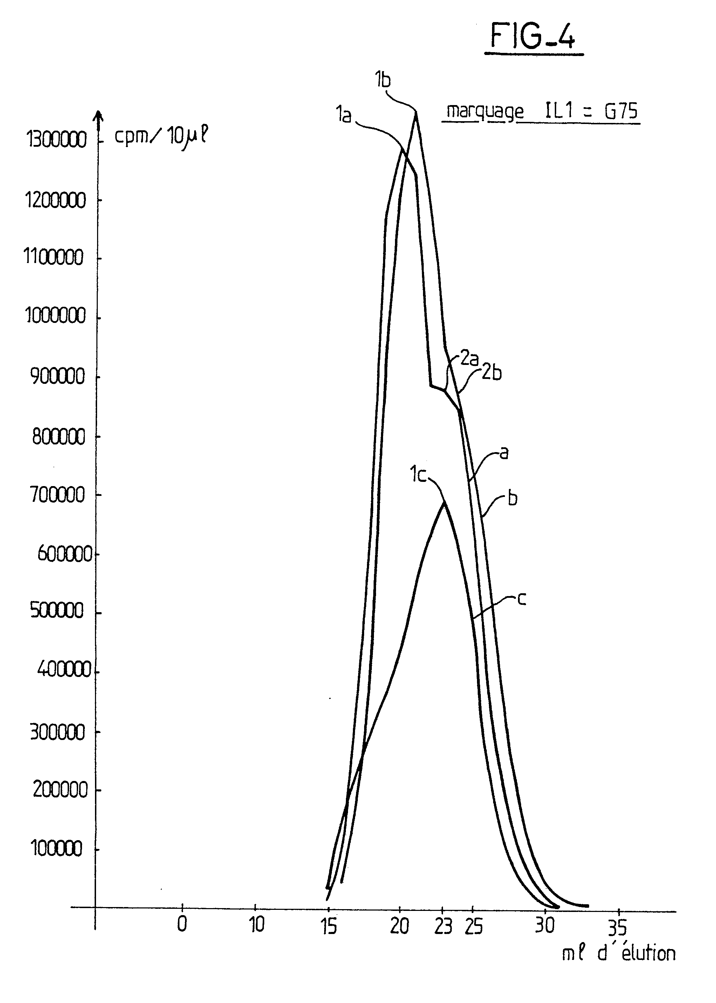

. la figure 4 représente des pics d'élution sur colonne Séphadex G75 de l'IL-1 marqué à I[125].

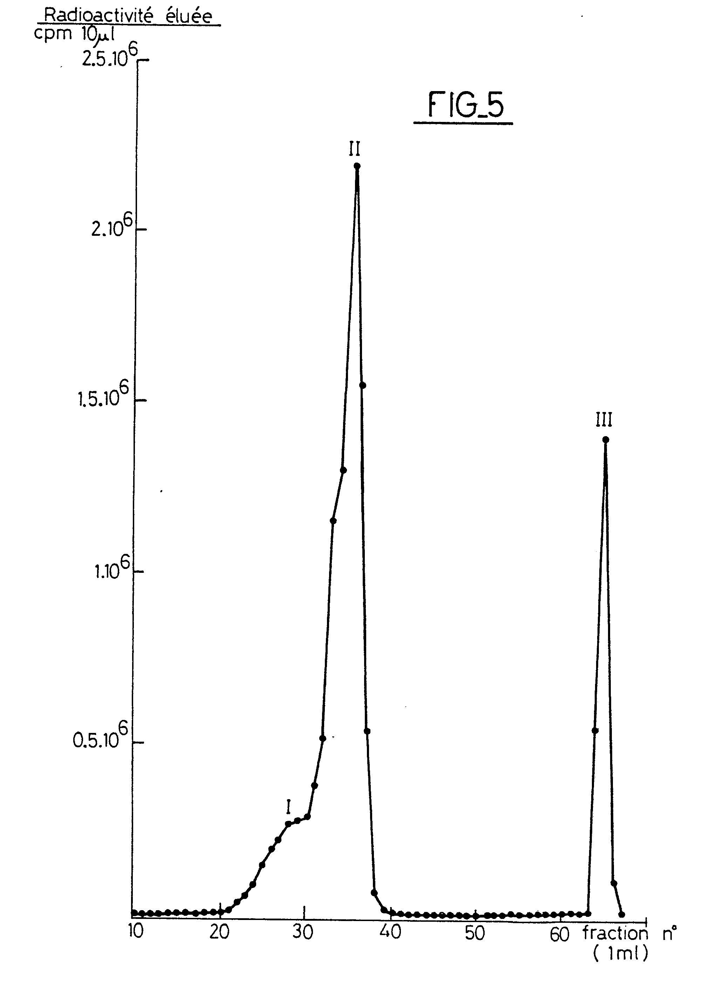

. La figure 5 représente des pics d'élution sur colonne ACa 34 sans addition de poly-L-lysine dans le tampon d'élution ;

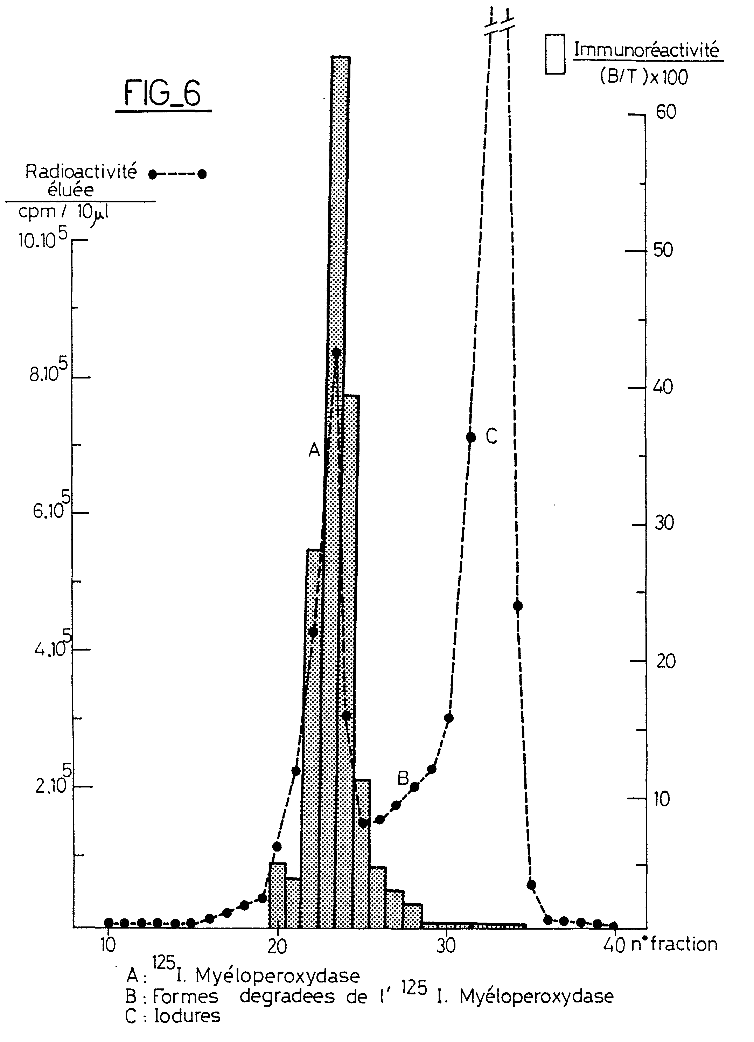

. La Figure 6 représente des pics d'élution sur colonne ACa 34 avec addition de poly-L-lysine (0,1 %) dans le tampon d'élution.

Dans la présente demande, on entend par :

- "tampon Tris" : un tampon Tris 40 mM pH 8 contenant 30 mM NaCl, 20 % de glycérol et 10 mM de dithiothréitol,

- "tampon d'incubation" : un tampon phosphate PO₄ 0,05 M pH 7,5 avec 5 ‰ de BSA et 0,5 ‰ de NaN₃,

- "tampon polylysine" : le même tampon d'incubation + 1 ‰ de polylysine (polylysine SIGMA, PM = 14 000 degré de polymérisation 70).

- "tampon éthylène glycol" : le tampon d'incubation + 15 % d'éthylène glycol.

Par la suite, on a utilisé un interféron γ humain (IFNγ-hu) (Gg 23-901-530) du National Institutes of Health Bethesda (N.I.H.) comme référence pour les dosages radioimmunologiques. Cet IFNγ (N.I.H) d'origine non recombinante est en solution dans un milieu de culture enrichi d'albumine humaine et ne connaît donc pas les problèmes liés aux phénomènes d'adsorption.

EXEMPLE I : Influence de la polylysine sur l'adsorption de l'interféron γ.

Cette influence a été appréciée lors de dosages radioimmunologiques (A) et lors de la purification de l'interféron γ marqué (B).

### (A) Dosages radioimmunologiques

Comme traceur, ainsi que pour l'immunisation, on a utilisé de l'interféron γ recombinant produit par culture de lignée cellulaire d'ovaire de hamster chinois-avec des plasmides codant pour de l'IFNγ-hu et de l'ADNc de dihydrofolate réductase de souris selon (3) et(4) . Il était dilué dans du tampon Tris.

Cet interféron IFNγ-hu a été marqué avec I[125] à l'aide de chloramine T selon la méthode de Green Wood et al. (1). La séparation de IFNγ-hu marqué des radicaux radioactifs a été effectuée sur colonne Séphadex G25 (0,9 x 30 cm) avec un tampon d'élution correspondant au "tampon d'incubation + 15 % d'éthylène glycol"(= "tampon éthylène glycol") ou en "tampon polylysine".

Le produit marqué était élué dans le premier pic principal. Le pic principal de radioactivité était recueilli dans une zone d'élution correspondant environ à 40-50 KD. Ce pic a été utilisé comme traceur dans les radioimmunodosages.

On a utilisé pour ces dosages un antisérum polyclonal de lapin.

Un lapin a reçu une émulsion constituée de 0,5 ml d'adjuvant complet de Freund et 0,5 ml de NaCl 0,9 % contenant 7,5 à 10.10⁵U de IFNγ-hu. Selon la méthode de Vaitukaitis (2), l'immunisation a consisté en 10 injections intradermales sur chaque côté de l'épine dorsale. Cinq administrations de rappel ont été effectuées à six semaines d'intervalle en utilisant la même quantité de IFNγ-hu. Quatorze jours après le dernier rappel, l'inverse de la dilution d'antisérum permettant d'obtenir une liaison de 30 % de l'antigène marqué était de 60 000.

### Méthode de dosage :

La première étape consiste dans l'incubation de 0,1 ml du tampon d'incubation contenant soit des quantités croissantes d'IFNγ non marqué, soit l'échantillon à doser et 0,1 ml d'antisérum à la dilution initiale de 1/60 000. Le volume total (0,2 ml) était incubé à 4°C pendant 24 heures.

Le traceur (dans 0,1 ml de tampon contenant du sérum de lapin normal à dilution 10⁻²) était ajouté au milieu pour une incubation supplémentaire de 24 heures.

L'IFNγ-hu marqué libre était séparé de la partie liée à l'anticorps par double précipitation. Au milieu d'incubation était ajouté 1 ml de tampon d'incubation + 6 % de polyéthylène glycol (PEG 6000), + 0,02 % (W/V) de cellulose microcristalline + 0,5 % de tween 20 et + de l'antisérum anti-lapin préparé chez le mouton dilué à 1:150. Après 20 minutes d'incubation et 15 minutes de centrifugation, le surnageant était mis à l'écart et le précipité était compté.

La figure 1 représente des courbes semi-logarithmiques stardard de dosage, selon la méthode décrite ci-dessus pour différents produits (S₁ à S₄ ) :

. l'IFN γ-hu de référence (N.I.H.) dans le "tampon d'incubation" : S₁,

. l'IFNγ-hu recombinant (AMERSHAM) non glycosylé,

a) liquide dans le "tampon d'incubation" : S₂ ,

b) le même qui a été congelé deux semaines dans le même tampon, puis décongelé juste avant l'incubation : S₃,

c) le même AMERSHAM qui a été lyophylisé dans des fioles siliconées avant réhydration dans le même tampon : S₄.   1) On observe sur la figure 1 qu'en "tampon d'incubation", soit par congélation (S₃) ou par

lyophilisation ($S_4$), il y a une perte d'immunoréactivité du produit AMERSHAM par rapport à la référence ($S_1$) qui ne connaît pas d'adsorption (IFN$\gamma$ N.I.H. produit froid et antisérum conservé congelé).

En effet, sur cette figure B représente la radioactivité du précipité, c'est-à-dire de l'IFN$\gamma$ marqué lié à l'anticorps spécifique, T représente la radioactivité totale du traceur initial.

Pour une valeur donnée de B, il faut des quantités supérieures d'IFN$\gamma$ AMERSHAM par rapport à la référence, que ce soit pour le produit conservé dans "tampon d'incubation" ou les produits qui ont été congelés et lyophylisés. Il y a donc une perte d'immunoréactivité de l'antigène lors de l'incubation pour le dosage. On perd de l'antigène froid par adsorption sur le flacon lors de la conservation.

En revanche, figure 2, les produits AMERSHAM non glycosylés ont été mis en solution dans un "tampon polylysine", les différentes courbes :

$S_1'$     NIH (Référence) (produit froid)

$S_2'$     liquide (AMERSHAM)

$S_3'$     congelé (AMERSHAM)

$S_4'$     lyophylisé (AMERSHAM)

montrent qu'il n'y a pas perte d'immunoréactivité par rapport au NIH. La différence existant entre la référence N.I.H. et les standards AMERSHAM est due à un étalonnage différent.    2) On a réalisé des courbes RIA (figure 3)

a) en ajoutant de la polylysine lors de l'incubation seulement où se produit la réaction antigène-anticorps spécifique et

b) sans en ajouter, pour un même standard dans les deux cas (AMERSHAM ou NIH).

On obtient des courbes quasiment superposées, ce qui indique donc que la polylysine ne perturbe pas la réaction antigène-anticorps spécifique.

Sur la figure 3, les courbes $S_1''$ à $S_4''$    représentent

$S_1''$    : standard NIH en "tampon d'incubation",

$S_2''$    : standard NIH en "tampon polylysine",

$S_3''$    : standard AMERSHAM en "tampon d'incubation",

$S_4''$    : standard AMERSHAM en "tampon polylysine",

$B_0$   = radioactivité de l'antigène marqué, lié à l'anticorps en l'absence d'antigène froid,

B = radioactivité de l'antigène marqué, lié à l'anticorps spécifique à une dilution constante de ce dernier, et pour une quantité donnée d'antigène froid.

## B) Purification d'interféron $\gamma$ marqué

L'expérience a été réalisée avec de l'interféron $\gamma$ AMERSHAM.

Le tableau I ci-après montre qu'uniquement en "tampon polylysine" on élue pratiquement tout ce qui a été déposé sur une colonne de G25. Dans ce tableau I R représente ce qui reste accroché à la colonne.

On réalise une succession de prélèvement par fraction de 1 ml dans une série de tubes (1 à 11). Les molécules de plus petites tailles (ici $I^{125}$) sont éluées en dernier.

Les tubes 7 et 8 représentent les tubes d'élution du traceur.

On compte 1500-2000 cpm en "tampon polylysine" pour le traceur et seulement 350 cpm pour ce qui reste accroché à la colonne.

## Tableau I

### (les valeurs sont données en cpm)

| | 5µl IFNγ 5µl PO₄ 0,5M *T. Ethylène glycole | 5µl IFNγ 5µl TRIS + mixture T. Ethyl. glycole | 5µl IFNγ 5µl PO₄ 0,5M ·Tampon polylysine |
|---|---|---|---|
| 1 | - | - | - |
| 2 | - | - | - |
| 3 | - | - | - |
| 4 | - | - | - |
| 5 | - | 15 | - |
| 6 | 250 | 15 | 15 |
| 7 | 1K5 | 1K | 2K |
| 8 | 300 | 800 | 1,5K |
| 9 | 100 | 100 | 150 |
| 10 | 100 | 100 | 150 |
| 11 | *R 1,5K | R 1,5K | R 350 |

\* T = tampon

R = reste

En outre, on a observé que 10µl d'une solution de polylysine à 1/1000 ne gênent pas lors du marquage.

EXEMPLE II : Influence de la polylysine sur la fixation non spécifique de l'interféron γ.

Cette fixation non spécifique s'apprécie en mesurant $T_o$ qui représente le "témoin", c'est-à- dire la radioactivité représentée par la quantité de produits marqués, liés à l'anticorps de précipitation qu'on rajoute pour précipiter le complexe formé par l'antigène marqué avec l'anticorps spécifiques. $T_o$ se mesure en introduisant uniquement l'anticorps précipitant (antisérum antiglobuline de lapin préparé chez le mouton) et l'antigène marqué et en séparant le précipité obtenu.

Dans les tableaux II et III ci-après, on a également mesuré AT, $B_o$, B2U et B20U.

AT représente la radioactivité totale initiale du traceur.

$B_o$ représente la radioactivité du traceur lié à l'anticorps spécifique en l'absence d'antigène froid.

B2U et B20U représentent la radioactivité de l'antigène marqué, lié à l'anticorps spécifique en présence de 2U et 20U d'antigène froid.

Comme traceur, on a utilisé plusieurs solutions de marquage, soit directement après marquage, soit après purification sur colonne Séphadex G25 :

APRES MARQUAGE:

signifiant que l'on a utilisé 20 µl de la solution de marquage +1 ml de "tampon d'incubation" ou 1 ml de "tampon polylysine", et

APRES G25:
signifiant que l'on a utilisé 30 μl de la solution de marquage appliquée sur colonne Séphadex G25 (H = 30 cm diamètre = 0,8 cm) et éluée en "tampon incubation + 15 % éthylène glycol" ("tampon éthylène glycol") ou en "tampon polylysine").

La solution A provient d'une solution de marquage constituée par :
- 5 μl de IFNγ AMERSHAM L-I3
- 5 μl de $PO_4$ 0,5 M
- 10 μl de $I^{125}$
- 10 μl de chloramine T (20 μg, 30 secondes)
- 10 μl de métabisulfite (20 μg)
- 300 μl de $PO_4$ 0,05 M.

La solution B = même solution que A, mais avec 5 μl de Tris 0,5 M au lieu de 5 μl de $PO_4$ 0,5 M.

La solution C = la solution A, mais le tampon $PO_4$ est additionné de polylysine ( "tampon polylysine").

La solution D = la solution A, mais avec 2,5 μl de IFNγ BOEHRINGER au lieu de 5 μl de IFNγ AMERSHAM.

On a réalisé deux séries de dosages, un dosage n° 1 avec des incubations en "tampon incubation" d'une journée à 4°C et un dosage n° 2 avec des incubations en "tampon incubation" de 4 heures à 20°C,au tableau II. Au tableau III, on a également effectué deux dosages supplémentaires avec des incubations en "tampon polylysine".

Au tableau III, les valeurs soulignées sont celles correspondant à des incubations en"tampon polylysine".

Les tableaux II et III ci-après montrent que les produits marqués, lorsqu'ils sont conservés en "tampon incubation" ou lorsqu'ils sont conservés et élués en "tampon éthylène glycol" et incubés en "tampon incubation" donnent un "témoin" fort important et une sensibilité anormalement élevée par rapport à nos résultats obtenus avec le traceur glycosylé de l'EXEMPLE I A) qui étaient de l'ordre de 1,5 à 2. En revanche, ces produits marqués, lorsqu'ils sont conservés et éventuellement élués en "tampon polylysine" et incubés en "tampon incubation" donnent un "témoin" nettement plus faible et une sensibilité qui se rapproche de celle donnée par le traceur glycosylé de référence.

Enfin, les résultats des dosages n° 3 et 4 du tableau III montrent que les produits marqués conservés et incubés en "tampon polylysine" donnent un "témoin" encore plus faible que celui obtenu avec les mêmes produits incubés en "tampon incubation".

Des résultats similaires ont été obtenus avec l'IFNγ AMERSHAM et BOEHRINGER.

Par conséquent, la réduction de la fixation non spécifique à l'aide de polylysine est bien mise en évidence.

# Tableau II

| Solution de marquage | | | | | |
|---|---|---|---|---|---|
| Solution A avec : <br><br>Après marquage = conservé en "tampon d'incubation" <br><br>Après G25 = élué et conservé en "tampon éthylène glycol" | | Solution B avec : <br><br>Après marquage = conservé en "tampon d'incubation" <br><br>Après G25 = élué et conservé en "tampon éthylène glycol" | | Solution C avec : <br><br>Après marquage = conservé en "tampon polylysine" <br><br>Après G25 = élué et conservé en "tampon polylysine" | |
| Après marquage | Après G25 | Après marquage | Après G25 | Après marquage | Après G25 |
| DOSAGE N° 1 - 1 J à 4°C - INCUBE EN "TAMPON INCUBATION" | | | | | |
| $T_o$   16,3 | 18,0 | 16,5 | 17,1 | 5,9 | 7,7 |
| AT   32 600 | 32 250 | 32 700 | 40 500 | 34 800 | 32 300 |
| Bo   **29,6** | 28,0 | 27,5 | 25,4 | 34,1 | 36,8 |
| B2U   **72,0** | 79,0 | 75,2 | 77,1 | 90,0 | 85,8 |
| B10U   23,6 | 21,0 | 24,3 | 22,4 | 47,5 | 47,2 |
| DOSAGE N° 2 - 4 H à 20°C - INCUBE EN "TAMPON INCUBATION" | | | | | |
| $T_o$   13,8 | 14,6 | 14,3 | 14,7 | 4,8 | 7,6 |
| AT   33 400 | 32 600 | 32 400 | 40 800 | 35 500 | 33 150 |
| Bo   23,1 | 23,1 | 23,3 | 22,1 | 25,1 | 27,4 |
| B2U   67,0 | 69,6 | 66,0 | 64,7 | 92,8 | 84,6 |
| B20U   23,3 | 23,8 | 22,7 | 21,7 | 56,1 | 47,0 |

## Tableau III

| Solution de marquage | | | | | |
|---|---|---|---|---|---|
| Solution A avec : Après marquage = conservé en "tampon d'incubation" Après G25 = élué et conservé en "tampon éthylène glycol" | | Solution A avec : Après marquage = conservé en "tampon polylysine" Après G25 = élué et conservé en "tampon polylysine" | | Solution D avec : Après marquage = conservé en "tampon polylysine" Après G25 = élué et conservé en "tampon polylysine" | |
| Après marquage | Après G25 | Après marquage | Après G25 | Après marquage | Après G25 |
| DOSAGE N° 3 - 1 JOUR A 4°C - INCUBE EN "TAMPON INCUBATION" OU "TAMPON POLYLYSINE" | | | | | |
| $T_0$ 3,1 21,6 | 2,5 15,8 | 2,1 5,4 | 2,8 5,8 | 1,5 2,0 | 1,4 2,4 |
| AT 34 300 32 800 | 33 800 30 400 | 32 900 36 300 | 29 500 27 140 | 33 000 34 500 | 41 000 38 000 |
| BO 35,7 25,3 | 38,8 30,2 | 36,7 34,9 | 22,7 26,7 | 36,8 36,0 | 93,9 35,2 |
| DOSAGE N° 4 - 4 H A TEMPERATURE ORDINAIRE "TAMPON INCUBATION" OU "TAMPON POLYLYSINE" | | | | | |
| $T_0$ 3,0 20,4 | 2,4 18,2 | 2,0 5,1 | 2,7 5,6 | 1,5 2,2 | 1,6 2,0 |
| AT 34 900 33 400 | 30 800 30 300 | 33 400 36 000 | 29 800 26 600 | 33 800 34 200 | 41 000 38 500 |
| BO 24,8 17,2 | 29,0 16,3 | 25,6 25,3 | 14,5 22,0 | 27,6 27,5 | 26,7 27,9 |

Les valeurs soulignées sont obtenues pour des incubations en "tampon polylysine".

EXEMPLE III : Influence de la concentration en polylysine, du poids moléculaire et degré de polymérisation de la polylysine.

Nous avons testé des concentrations de plus en plus faibles de polylysine de poids moléculaire et de degré de polymérisation différents, ainsi que le bromure d'alkyl-triméthylammonium qui est un détergent très puissant.

1) Pour le Tableau IV, ci-après, l'interféron γ marqué AMERSHAM non purifié sur gel de Séphadex et conservé en "tampon éthylène glycol" est incubé dans le tampon habituel, ainsi que dans du tampon contenant différentes polylysines ou détergents à des concentrations de plus en plus faibles (0,05 ‰ à 1 ‰).

La polylysine de poids moléculaire (P.M.) 14000 et de degré de polymérisation (D.P.) 70 nous donne les résultats les plus satisfaisants, même jusqu'à une concentration de 0,05 ‰ puisqu'on obtient les valeurs de $T_0$ les plus faibles par rapport aux polylysine de poids moléculaires supérieurs.

2) Le Tableau V, ci-après, met également en évidence la spécificité de la polylysine de P.M. 14000 et D.P. 70 puisqu'il y apparaît que la lysine ou une polylysine de P.M. et D.P. plus faible donne également de moins bons résultats. Ils ont en effet été testés lors du marquage dans le tampon de conservation ou d'élution du produit marqué.

Le Tableau V montre que, juste après marquage, quel que soit le tampon de conservation utilisé, la dilution de l'antigène marqué pour obtenir environ 30 000 cpm/100 µl est identique. En revanche, après élution sur colonne G25, les résultats sont différents suivant le tampon utilisé. En effet, en présence d'éthylène glycol il faut diluer l'antigène marqué 500 fois, en présence de polylysine P.M. 14000 et D.P. 70 il faut diluer l'antigène marqué 3000 fois, en présence de polylysine P.M. 360 et D.P. 17 il faut diluer 2000 fois et, enfin, avec de la lysine il faut diluer 750 fois.

Pour le tableau V, un IFNγ BOEHRINGER marqué et purifié sur gel de Séphadex a été utilisé.

## Tableau IV

| 1 JOUR A 4°C | "TAMPON INCUBA-TION" | "TAMPON POLYLYSINE" Degré de polymérisation : 70 PM : 14.000 | | | "TAMPON POLYLYSINE" Degré de polymérisation : 220 PM : 40.000 | | | "TAMPON POLYLYSINE" Degré de polymérisation : 1850 PM : 390.000 | | | ALKYLTRI-METHYL-AMMONIUM BROMIDE | | |
|---|---|---|---|---|---|---|---|---|---|---|---|---|---|
| | | 1‰ | 0,5‰ | 0,1‰ | 1‰ | 0,5‰ | 0,1‰ | 1‰ | 0,5‰ | 0,1‰ | 1‰ | 0,5‰ | 0,1‰ |
| AT | 29.389 | 23.711 | 34.178 | 27.799 | 32.685 | 30.124 | 30.779 | 32.210 | 32.423 | 31.967 | 34.900 | 31.200 | 32.039 |
| T₀ | 9,0 | 3,3 | 3,3 | 3,7 | 22,1 | 21,0 | 9,7 | 6,9 | 5,4 | 4,1 | 7,1 | 9,6 | 8,0 |
| BO – T₀ | 30,8 | 33,6 | 30,3 | 32,7 | 38,2 | 26,6 | 32,0 | 29,5 | – | – | 1,7 | 8,9 | 30,0 |
| 24/2/1987 | | 0,1‰ | 0,05‰ | 0,01‰ | 0,1‰ | 0,05‰ | 0,01‰ | 0,1‰ | 0,05‰ | 0,01‰ | 0,1‰ | 0,05‰ | 0,01‰ |
| AT | 30.600 | 27.255 | 29.040 | 52.174 | 23.487 | 24.915 | 28.446 | – | – | – | 27.666 | 30.423 | 26.237 |
| T₀ | 11,5 | 4,4 | 4,1 | 4,8 | 9,9 | 8,4 | 7,5 | – | – | – | 7,6 | 7,8 | 9,6 |
| BO – T₀ | 26,9 | 29,5 | 29,6 | 22,5 | 32,1 | 30,9 | 30,6 | – | – | – | 29,5 | 29,8 | 29 |

Tableau V

0 296 050

| | MARQUAGE IFNγ BOEHRINGER | | | | | | | | INCUBATION 1 JOUR A 4°C | | | | | | | |
|---|---|---|---|---|---|---|---|---|---|---|---|---|---|---|---|---|
| | Conservation et Elution dans "TAMPON ETHYLENE GLYCOL" | | | | Conservation et Elution dans "TAMPON POLYLYSINE" 1 ‰ DP : 70 - PM : 14000 | | | | Conservation et Elution dans "TAMPON POLYLYSINE" 0,5 ‰ DP : 17 - PM 3600 | | | | Conservation et Elution dans "TAMPON LYSINE" 1 ‰ | | | |
| | Incubation dans "Tampon incubation" | | Incubation dans "Tampon élution" | | Incubation dans "Tampon incubation" | | Incubation dans "Tampon élution" | | Incubation dans "Tampon incubation" | | Incubation dans "Tampon élution" | | Incubation dans "Tampon incubation" | | Incubation dans "Tampon élution" | |
| | Après marquage | G25 | Après marquage | G25 | Après marquage | G25 | Après marquage | G25 | Après marquage | G25 | Après marquage | G25 | Après marquage | G25 | Après marquage | G25 |
| AT | 27.955 | 33.621 | 27.866 | 34.300 | 26.594 | 29.760 | 28.400 | 33.900 | 33.000 | 32.400 | 31.000 | 35.895 | 30.100 | 30.300 | 32.564 | 36.100 |
| $T_0$ | 2,5 | 2,4 | 2,9 | 2,9 | 2,1 | 4,7 | 1,9 | 2,1 | 2,5 | 6,3 | 1,7 | 2,6 | 2,6 | 6,7 | 2,2 | 6,3 |
| $B_0+T_0$ | 46,1 | 40,8 | 44,1 | 37,2 | 47,1 | 35,0 | 43,4 | 33,9 | 42,4 | 28,0 | 41,0 | 32,1 | 45,0 | 25,4 | 41,7 | 29,0 |
| B 5U | 49,0 | 47,0 | 56,0 | 54,0 | 50,7 | 55,1 | 52,5 | 50,1 | 53,7 | 57,6 | 53,5 | 51,7 | 47,7 | 59,0 | 53,0 | 56,0 |
| Dilution de l'antigène marqué | 1/250 (20 µl → 5 ml) | 1/500 (10 µl → 5 ml) | 1/250 | 1/500 | 1/250 (20 µl → 5 ml) | 1/3000 (10 µl → 30 ml) | 1/250 | 1/3000 | 1/250 (20 µl → 5 ml) | 1/2000 (10 µl → 20 ml) | 1/250 | 1/2000 | 1/250 (20 µl → 5 ml) | 1/750 (10 µl → 7,5 ml) | 1/250 | 1/750 |

EXEMPLE IV : Influence de la polylysine sur d'autres substances.

A) TNFα

L'élution sur colonne Séphadex G50 du TNFα marqué est identique en tampon enrichi en BSA (10 g/l) ou en "tampon polylysine".

Les résultats obtenus avec le TNFα marqué non purifié sur Séphadex G50 et conservé en tampon contenant 10 g d'albumine sont non reproductibles, par contre, ceux obtenus en conservant le TNFα marqué dans du tampon polylysine sont tout à fait satisfaisants. Après élution sur colonne G50, les résultats sont semblables, quel que soit le tampon employé. Par conséquent, même si la polylysine n'est pas indispensable, sa présence ne diminue en rien les résultats obtenus, et, plutôt, les améliore.

B) IL-1

1) Sur la figure 4 sont représentés les pics d'élution de l'IL-1 avec différents tampons d'élution (a, b et c).

Les pics 1a et 2a sont obtenus en marquant avec 20 μg de chloramine T + 20 μg de métabisulfite et en éluant avec un "tampon éthylène glycol".

Les pics 1b et 2b sont obtenus avec le même marquage, mais en éluant en "tampon polylysine".

Les pics 2a et 2b sont en fait des épaulements.

a = 20 μg chloramine T + 20 μg métabisulfite
"Tampon éthylène glycol"
b = 20 μg chloramine T + 20 μg métabisulfite
"Tampon polylysine"
c = 20 μg chloramine T
"Tampon polylysine".

La dégradation d'un produit s'apprécie par rapport à la symétrie du pic d'élution.

Comme le montre le profil d'élution de la figure 4 sur colonne Séphadex G75, c'est l'IL-1 marquée en présence de 2 μg de chloramine T et éluée en "tampon polylysine"(pic 1c)qui semble la moins dégradée. En effet, d'après nos expériences précédentes, seule la fraction éluée au niveau du tube 23 donne des résultats satisfaisants tant au point de vue de la fixation (à l'anticorps spécifique), qu'au point de vue de la sensibilité (immunoréactivité).

2) Sur le Tableau VI ci-après, on peut constater que l'IL-1 marquée en présence de 2 μg de chloramine T et éluée en "tampon polylysine" donne bien les meilleurs résultats : en effet, le témoin $T_o$ est faible, le pourcentage de fixation très bon ($B_o$) et la sensibilité correcte (B 200 ng).

ANTISERUM BIOGEN 1/1500　　　　　　　　　　　　　　　　　　　　TAMPON INCUBATION

| | ETUDE DES MARQUAGES D'IL-1 | | | | | | | | | | |
|---|---|---|---|---|---|---|---|---|---|---|---|
| | MARQUAGE 2 μg CHLORAMINE T - élué en TAMPON POLYLYSINE | | | MARQUAGE 20 μg CHLORAMINE METABISULFITE-TAMPON ETHYLENE GLYCOL (élution) | | | | MARQUAGE 20 μg CHLORAMINE METABISULFITE-TAMPON POLY-LYSINE (élution) | | | |
| Expérience 1 | Après marquage | Après G25 | Après G25 PIC 1C | Après marquage | Après G25 | Après G75 PIC 1a | Après G75 PIC 2a | Après marquage | Après G25 | Après G75 PIC 1b | Après G75 PIC 2b |
| AT | 22.815 | 28.998 | 22.750 | 20.060 | 34.100 | 36.600 | 19.500 | 23.100 | 27.100 | 27.600 | 24.200 |
| $T_o$ | 1,5 | 1,6 | 1,4 | 1,5 | 1,2 | 1,7 | 1,9 | 1,4 | 1,6 | 1,2 | 1,4 |
| Bo+$T_o$ | 12,8 | 32,4 | 21,3 | 54,7 | 52,4 | 68,7 | 30,6 | 52,3 | 55,5 | 72,5 | 27,3 |
| Expérience 2 | | | | | | | | | | | |
| AT | 21.232 | 27.000 | 20.700 | 21.062 | 21.700 | 20.540 | 20.653 | 23.700 | 27.350 | 25.232 | 22.876 |
| $T_o$ | 1,6 | 1,3 | 1,4 | 1,5 | 1,5 | 1,4 | 1,4 | 1,7 | 1,6 | 1,7 | 1,4 |
| Bo+$T_o$ | 12,3 | 32,9 | 21,8 | 42,9 | 49,2 | 63,8 | 25,0 | 48,3 | 49,8 | 62,5 | 25,9 |
| B200 ng % | 83,4 | 78,5 | 62,2 | 93,2 | 87,8 | 93,9 | 78,6 | 90,9 | 93,3 | 92,1 | 81,0 |

| ANTISERUM IL-1 62 1/25000 ( IRE-MEDGENIX ) | | | | | | | | | | | |
|---|---|---|---|---|---|---|---|---|---|---|---|
| Expérience 2 | | | | | | | | | | | |
| AT | 20.500 | 27.140 | 20.600 | 20.900 | 21.600 | 20.499 | 20.231 | 24.600 | 26.980 | 24.600 | 23.260 |
| $T_o$ | 1,4 | 1,5 | 1,2 | 1,3 | 1,4 | 1,4 | 1,3 | 1,8 | 1,4 | 1,6 | 1,3 |
| Bo+$T_o$ | 14,5 | 31,1 | 29,9 | 25,9 | 29,6 | 32,5 | 26,2 | 27,8 | 31,0 | 31,6 | 24,2 |
| B200ng % | 44,1 | 39,2 | 16,3 | 68,3 | 67,2 | 86,7 | 28,6 | 69,0 | 69,0 | 81,6 | 23,6 |

Tableau VI

0 296 050

## C) MYELOPEROXYDASE

Grâce à la polylysine, on a pu éluer la myéloperoxydase qui, comme l'IFNγ, restait très fortement accrochée à une colonne ACA 34.

De la même manière que pour l'interféron γ AMERSHAM et BOEHRINGER, pour la myéloperoxydase, la polylysine est donc indispensable pour obtenir des résultats satisfaisants.

## UTILISATION DE LA POLY-L-LYSINE (SIGMA P-6516) DANS LA PURIFICATION DE L'$^{125}$I-MYELOPEROXYDASE GRANULOCYTAIRE HUMAINE

Après marquage, l'$^{125}$I-Myeloperoxydase ($^{125}$I-MPO) est purifiée sur Aca 34 (LKB).

Il ressort de la figure 5 que les premiers essais d'élution en tampon phosphate 0,05 M pH 7,5 (avec 0,5 % de sérum albumine bovine et 0,05 % d'azide de Na) ont été infructueux : l'$^{125}$I-MPO n'était éluée qu'après plus de 60 fractions de 1 ml, c'est-à-dire plus de 10 heures d'élution, et fortement dégradée. Les tests d'immunoréactivité ont montré une fixation non spécifique très élevée (25 à 30 %) et une dégradation complète du traceur après 15 jours.

Sur la figure 1 :

I correspond aux fragments dégradés de l'$^{125}$I-Myéloperoxydase

II correspond aux iodures

III correspond à l'$^{125}$I-Myéloperoxydase

Il ressort de la Figure 6 que l'addition de 0,1 % de poly-L-lysine au tampon d'élution a permis d'éluer l'$^{125}$I-Myeloperoxydase à l'endroit attendu en fonction de son poids moléculaire (après 2 à 3 heures d'élution). Le traceur ainsi obtenu présente une excellente immunoréactivité (jusqu'à 60 % de fixation pour un antisérum dilué à 1/24.000) avec une fixation spécifique faiblz (maximum : 4 %) et une très faible dégradation au cours du temps (le traceur reste utilisable jusqu'à 40 jours après marquage).

Sur la Figure 2 :

A correspond à l'$^{125}$I-myeloperoxydase

B correspond aux formes dégradées de l'$^{125}$I-myeloperoxydase

C correspond aux iodures.

De manière générale, pour les autres substances étudiées, même lorsqu'elle n'est pas indispensable, la présence de polylysine ne diminue en rien les résultats obtenus ou mieux les améliore.

## REFERENCES BIBLIOGRAPHIQUES

1) GREENWOOD, F., HUNTER, W., GLOVER, S. et al. : Preparation of I.125 labeled human growth hormone of high specific radioactivity. Biochem. J., 8 : 114-120, 1963.

2) VAITUKAITIS, J., ROBBINS, JB., NIESCHLAG, E. et al. : A method for producing specific antisera with small doses of immunogens. J. Clin. Endocrinol. Metab., 33 : 988-991, 1971.

3) DEVOS, R., CHEROUTRE, H., TAYA, Y. et al. : Molecular cloning of human immune interferon cDNA and its expression in eukariotic cells. Nucleic. Acid. Res., 10 : 2487-2501, 1982.

4) SCAHILL, S., DEVOS, R., VAN DER HEYDEN, J. et al. : Expression and characterization of a human immune cDNA gene in chinese hamster ovary cells. Proc. Natl. Acad. Sci. USA, 80 : 4654-4659, 1983.

## Revendications

1. Solution d'une première substance de nature polypeptidique, utile notamment pour réduire les phénomènes d'adsorption de ladite substance, caractérisée en ce que la solution comporte notamment , outre ladite première substance, une seconde substance choisie parmi les amino-acides basiques monomères ou polymérisés.

2. Solution selon la revendication 1, caractérisée en ce que la solution comporte de la polylysine.

3. Solution selon la revendication 2, caractérisée en ce que la polylysine est de poids moléculaire 14000 et de degré de polymérisation 70.

4. Solution selon l'une des revendications 1 à 3, caractérisée en ce que ladite seconde substance est présente dans des dilutions pouvant descendre jusqu'à 0,05 %₀.

5. Solution selon l'une des revendications 1 à 4, caractérisée en ce que ladite seconde substance est présente dans des dilutions pouvant descendre jusqu'à 0,01 %₀.

6. Solution selon l'une des revendications précédentes, caractérisée en ce que ladite première substance de nature polypeptidique est marquée, notamment avec de l'iode radioactif.

7. Solution selon l'une des revendications 1 à 6, caractérisée en ce que ladite première substance de nature protéique est choisie notamment parmi l'interféron γ, le TNFα, l'IL-1, l'IL-2, le glucagon, l'insuline et la myéloperoxydase.

8. Solution selon l'une des revendications précédentes, caractérisée en ce que la solution est un tampon d'élution classique pour la purification de ladite première substance marquée ou non, à travers une colonne de purification, tampon auquel on a rajouté ladite seconde substance.

9. Solution selon l'une des revendications 1 à 7, caractérisée en ce que la solution est un tampon de conservation classique de ladite première substance marquée ou non, tampon auquel on a rajouté ladite seconde substance.

10. Solution selon l'une des revendications 1 à 7, caractérisée en ce que la solution est un tampon d'incubation de ladite première substance marquée ou non, antigène ou anticorps, destiné à des dosages immunologiques de ladite première substance, tampon auquel on a rajouté ladite seconde substance.

11. Solution selon l'une des revendications 1 à 10, caractérisée en ce que la solution ne comporte pas d'agent stabilisateur protéique tel que la BSA autre que ladite seconde substance.

12. Trousse de dosages immunologiques, caractérisée en ce qu'elle comporte des flacons contenant des solutions ou des lyophilisats de solutions selon l'une des revendications précédentes.

13. Trousse de dosages immunologiques d'une substance choisie parmi l'interféron γ, l'interleukine 1, la myeloperoxydase, caractérisée en ce qu'elle comporte des flacons contenant ladite substance marquée ou non, et un antisérum dirigé contre ladite substance marqué ou non, en solution dans des tampons selon l'une des revendications précédentes ou lyophilisé à partir de ces solutions.

029605C

FIG_1

029605C

## FIG-2

## FIG_3

FIG_4

marquage IL1 = G75

FIG_5

FIG_6

0296050

A : $^{125}$I. Myéloperoxydase
B : Formes degradees de l'$^{125}$I. Myéloperoxydase
C : Iodures

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 572 901  (R.L. CERIANI et al.)<br>* En entier *<br>--- | 1,2,6,7,9-13 | G 01 N  33/53<br>G 01 N  33/543<br>G 01 N  33/68 |
| A | EP-A-0 124 103  (MINNESOTA MINING AND MANUFACTURING CO.)<br>* Résumé; page 12, lignes 1-20; page 13, ligne 27 - page 15, ligne 13; page 29, line 12 - page 30; ligne 3 *<br>--- | 1,2,10,11 | |
| A | EP-A-0 140 489  (WAKO PURE CHEMICAL INDUSTRIES LTD)<br>* En entier *<br>--- | 1,11 | |
| A | CHEMICAL ABSTRACTS, vol. 100, no. 8, 20 février 1984; page 324, résumé no. 56788p, Columbus, Ohio, US; R. QUINN et al.: "Minimizing the aggregation of neutral insulin solutions", & J. PHARM. SCI. 1983, 72(12), 1472-1473<br>--- | 1,7,9 | |
| A | PATENT ABSTRACTS OF JAPAN, vol. 9, no. 79 (P-347)[1802], 9 avril 1985; & JP-A-59 210 366 (SUMITOMO KAGAKU KOGYO K.K.) 29-11-1984<br>* En entier *<br>----- | 1,10 | **DOMAINES TECHNIQUES RECHERCHES (Int. Cl.4)**<br><br>G 01 N |

Le présent rapport a été établi pour toutes les revendications

| Lieu de la recherche | Date d'achèvement de la recherche | Examinateur |
|---|---|---|
| LA HAYE | 21-07-1988 | HITCHEN C.E. |